# EUROPÄISCHE PATENTSCHRIFT

(11) **EP 1 346 710 B1**
(45) Veröffentlichungstag und Bekanntmachung des Hinweises auf die Patenterteilung: **25.10.2006**
(21) Anmeldenummer: 03006260.8
(22) Anmeldetag: 21.03.2003
(51) Int. Cl.: A61F 5/01

(54) **Sprunggelenkorthese**
Ankle joint orthesis
Orthèse pour la cheville

(30) Priorität: 22.03.2002 DE 10213238
(43) Veröffentlichungstag der Anmeldung: 24.09.2003
(73) Patentinhaber: Bauerfeind AG, 07937 Zeulenroda (DE)
(72) Erfinder: Reinhardt, Holger, 47906 Kempten (DE)
(74) Vertreter: Bardehle, Heinz

(56) Entgegenhaltungen:
- US-A- 4 977 891
- US-A- 5 125 400
- US-A- 5 348 530
- US-A- 5 389 065
- US-A- 5 489 259

## Beschreibung

Die Erfindung bezieht sich auf eine Sprunggelenkorthese mit zwei über die Knöchel den Unterschenkel entlang verlaufenden Schenkeln, die unter dem Fuß durch einen biegsamen Steg verbunden und mit ein Fluid enthaltenden Polsterkammern versehen sind.

Eine Sprunggelenkorthese gemäß dem Oberbegriff von Anspruch 1 ist aus der US-A-5 389 065 bekannt.

Eine Sprunggelenkorthese ist in der US-PS 5,125,400 dargestellt und beschrieben. Speziell behandelt werden in dieser Patentschrift Sprunggelenkorthesen, die pro Schenkel der Orthese jeweils mehrere über eigene Ventile getrennt aufblasbare Polsterkammern aufweisen, wobei ihre beiden Schenkel durch einen allein biegeelastischen Steg aus Klettverschlussmaterial verbunden sind. Darüber hinaus wird in der Patentschrift auch auf frühere Sprunggelenkorthesen verwiesen, die pro Schenkel jeweils mit nur einer Polsterkammer auskamen. Gemäß den Angaben in der Patentschrift führte jedoch die Verwendung von nur einer Polsterkammer zu hohen Drücken außerhalb des Bereichs des Knöchels.

Der Erfindung liegt die Aufgabe zugrunde, eine Sprunggelenkorthese mit ein Fluid enthaltende Polsterkammern zu schaffen, bei der der Knöchel einem besonders geringen Druck ausgesetzt ist und außerdem beim Gehen infolge der damit verbundenen rhythmischen Druckwirkung auf die Polsterkammern eine in Richtung Knie abnehmende Flächenpressung entsteht, durch die der Abfluss des Blutes aus dem Sprunggelenk gefördert wird. Letzteres ist vor allem dann von Bedeutung, wenn es sich um die Behandlung von Schwellungen im Bereich des Sprunggelenkes handelt.

Gelöst wird das Problem durch die Sprunggelenkorthese nach Anspruch 1.

Diese erfindungsgemäße Sprunggelenkorthese führt zunächst zu dem Vorteil einer vereinfachten Herstellung, da die Sprunggelenkorthese pro Schenkel mit jeweils nur einer Polsterkammer auskommt, so dass für die jeweilige Polsterkammer auch nur ein Einlassventil erforderlich ist. Die gewünschte unterschiedliche Flächenpressung wird trotz der Verwendung nur einer einzigen Polsterkammer pro Schenkel dadurch erreicht, dass jede Polsterkammer eine Längsunterteilung aufweist, die jeweils im Bereich des Knöchels die Zone reduzierter Ausdehnbarkeit schafft, da durch die Längsunterteilung in diesem Bereich die Polsterkammer eine Einbuchtung bildet, in die der Knöchel relativ druckfrei hineinragen kann. Dabei wird die Funktion der Polsterkammern beim Gehen und dabei erfolgendem Abrollen des Fußes in besonderer Weise ausgenutzt, nämlich dadurch, dass hierdurch ein rhythmisches Pumpen der Polsterkammern dadurch ermöglicht wird, dass der die beiden Schenkel der Sprunggelenkorthese verbindende Steg aus zugelastischem Material gestaltet ist. Der Steg kann sich beim Gehen mehr oder minder rhythmisch ausdehnen und zusammenziehen und bewirkt dabei ein entsprechendes Pumpen der Polsterkammern, ohne dass sich dabei besonders hohe Druckschwankungen ergeben, die durch den zugelastischen Steg, der sich dabei ausdehnt und zusammenzieht, weitgehend ausgeglichen werden.

Die Längsunterteilung wird in fabrikatorisch günstiger Weise durch zwischen gegenüberliegenden Wänden dieser Polsterkammer angeordnete Verbindungen hergestellt, die entlang der Längsunterteilung verlaufen. Diese Verbindungen werden zweckmäßig gemäß heute herrschender Technik durch Verschweißen gebildet. Es sei aber darauf hingewiesen, dass selbstverständlich die Verbindungen auch z.B. durch Kleben herbeigeführt werden können.

Eine einfache Gestaltung für die Verbindungen ergibt sich dadurch, dass diese durch in Reihe liegende Schweißpunkte gebildet sind. Andererseits ist es auch möglich, die Verbindungen durch Schweißnähte auszubilden.

Eine besonders günstige Gestaltung der Längsunterteilung ergibt sich dadurch, dass zu ihrer Realisierung zwei Strecken von Verbindungen vorgesehen sind, die derart V-förmig verlaufen, dass die beiden Strecken im Bereich des Knöchels zusammentreffen und in Richtung zum Knie so weit auseinander streben, dass, ausgehend vom Bereich des Knöchels, neben diesem zwei Unterkammern und im Übergang der Strecken in Richtung zum Knie drei nebeneinanderliegende Unterkammern gebildet sind, deren Querschnitt im gefüllten Zustand abnehmend geringer ist als der Querschnitt jeder der beiden Unterkammern im Bereich des Knöchels.

Bei dieser Gestaltung der Verbindungen ergeben sich im Bereich des Knöchels im Effekt zwei neben dem Knöchel liegende Unterkammern und im weiteren Bereich der Schenkel im Übergang der Strecken in Richtung zum Knie drei nebeneinander liegende Unterkammern, die alle Bestandteil der pro Schenkel einzigen Polsterkammer sind und somit beim Füllen der Polsterkammer mitgefüllt werden und sich hinsichtlich ihres Innendrucks in Richtung auf den gesamten Bereich der Polsterkammer auch ausgleichen können. Durch diese Gestaltung ergibt sich neben dem Knöchel ein Bereich mit zwei größeren Unterkammern und im Bereich am Ende des durch die Verbindungen gestalteten "V" drei nebeneinander liegende Unterkammern, deren Querschnitt im gefüllten Zustand geringer ist als der Querschnitt der beiden Unterkammern im Bereich des Knöchels. Wenn sich also beim Gehen ein Pumpeffekt auf die beiden Unterkammern im Bereich des Knöchels auswirkt, so geht von diesem ein Druck in Richtung auf die drei Unterkammern im Bereich des Endes des V aus, wo aber wegen der Verteilung des Drucks auf drei Unterkammern die Flächenpressung auf das Bein des Trägers abnimmt. Dadurch dass die beiden Strecken im Bereich des Knöchels zusammenlaufen und in Richtung zum Knie so weit auseinander streben, ergibt sich, dass, ausgehend vom Bereich des Knöchels neben diesem zwei Unterkammern und im Übergang der Strecken in Richtung zum Knie drei nebeneinanderliegende Unterkammern, deren Querschnitt im gefüllten Zustand abnehmend geringer ist als der Querschnitt jeder der beiden Unterkammern im Bereich des Knöchels, so dass hierdurch das Abströmen des Blutes aus dem Bereich des Knöchels wesentlich erleichtert wird. Dieser Effekt wird insbesondere auch dadurch ermöglicht, dass der Steg, der die beiden Schenkel der Sprunggelenkorthese unter dem Fuß verbindet, wegen seiner Zugelastizität beim Gehen sich ausdehnen und wieder zusammenziehen kann.

In den Figuren ist ein Ausführungsbeispiel der Erfindung dargestellt, es zeigen:
- Figur 1: eine an einem linken Fuß angebrachte Sprunggelenkorthese in perspektivischer Sicht;
- Figur 2: die gleiche Sprunggelenkorthese in einer Draufsicht auf das Schienbein;
- Figur 3: die Gestaltung gemäß Figur 2 um 90° gedreht;
- Figur 4: eine Polsterkammer im Schnitt mit durch Schweißpunkte gebildete Verbindungen;
- Figur 5: eine Polsterkammer im Schnitt mit durch Schweißnähte gebildete Verbindungen;
- Figur 6: einen Schnitt durch die Anordnung gemäß Figur 4 längs der Linie VI-VI;
- Figur 7: einen Schnitt durch die Anordnung gemäß Figur 4 längs der Linie VII-VII.

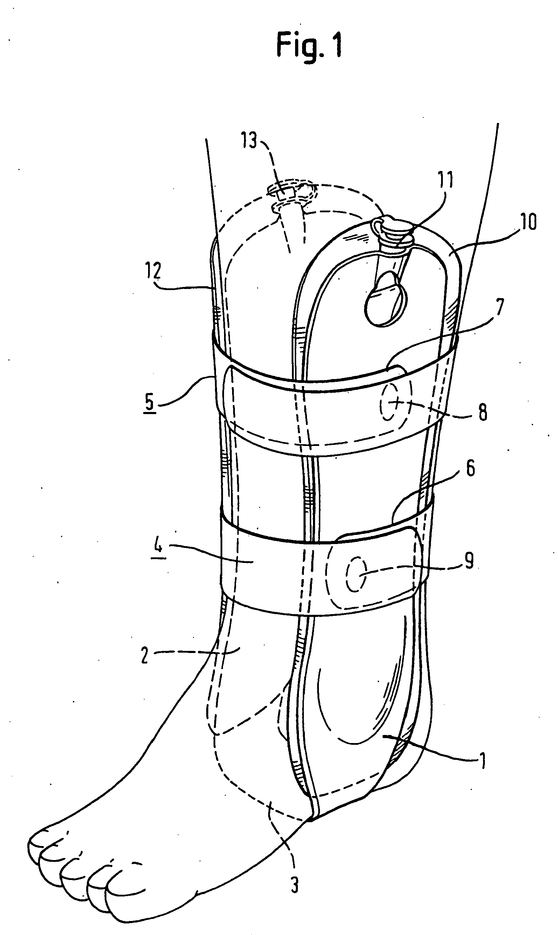

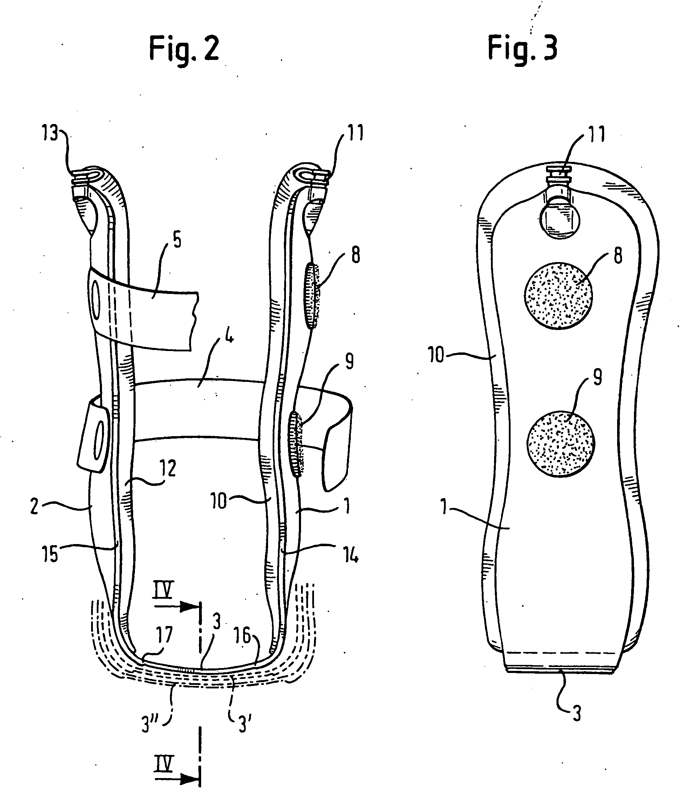

Die in der Figur 1 an einem Fuß angelegte Sprunggelenkorthese weist die beiden Schenkel 1 und 2 auf, die sich von der Fußsohle her in Richtung Knie erstrecken und im Bereich der Fußsohle durch den in Figur 1 unsichtbaren Steg 3 verbunden sind. Auf die Gestaltung und Funktion des Steges 3 wird im Zusammenhang mit der Figur 2 näher eingegangen. Zur Festlegung der Sprunggelenkorthese mit ihren beiden Schenkeln 1 und 2 dienen die beiden Gurte 4 und 5, die an dem Schenkel 2 festgelegt sind und sich im Bereich des Schenkels 1 überlappen. Der dabei unten liegende Teil 6 bzw. 7 ist mit einem Klettverschlussteil 8 bzw. 9 ausgestattet, das mit einem entsprechenden Teil auf der inneren Seite des überlappenden Endes des Gurtes 4 bzw. 5 in bekannter Weise zusammen wirkt. Diese Art der Festlegung einer mit zwei Schenkeln ausgestatteten Sprunggelenkorthese ist bekannt.

Der Schenkel 1 ist mit der durchgehenden Polsterkammer 10 verbunden, die bei angelegter Sprunggelenkorthese zwischen dem Schenkel 1 und dem Bein des Trägers liegt. Die Polsterkammer 10 wird über das Ventil 11 aufgeblasen, das in bekannter Weise gestaltet ist. Anstelle von Luft zum Aufblasen der Polsterkammer 10 kann natürlich auch ein anderes Fluid verwendet werden, jedoch sei darauf hingewiesen, dass für die Zwecke, wie hier beschrieben sind, in erster Linie Luft ein geeignetes Medium ist. Der auf der anderen Seite des Beins angeordnete Schenkel 2 trägt die Polsterkammer 12, die über das Ventil 13 gefüllt wird. Insofern liegen hier also bezüglich Schenkel 1 und Polsterkammer 2 spiegelbildliche Verhältnisse vor.

In der Figur 2 ist die Sprunggelenkorthese gemäß Figur 1 in einer Draufsicht auf das Schienbein des Trägers dargestellt, so dass die beiden Schenkel 1 und 2 direkt mit ihren Kanten 14, 15 sichtbar sind. Die Schenkel 1 und 2 werden im Bereich der Fußsohle, wie bereits im Zusammenhang mit Figur 1 beschrieben, durch den Steg 3 verbunden, der aus einem zugelastischen Material besteht. Die Schenkel 1 und 2 besitzen die Kanten 14 und 15, die in den Steg 3 übergehen. Der Steg 3 selbst ist, wie ersichtlich, dünner als die Kante 14 bzw. 15 ausgebildet, was an den beiden Schwächungsstellen 16 und 17 im Bereich des Übergangs des Stegs 3 in die Schenkel 1 und 2 ersichtlich ist. Bei dem Material des Stegs 3 kann es sich z.B. um ein thermoplastisches Elastomer handelt, das in das Material der Schenkel 1 und 2 über die beiden Schwächungsstellen 16 und 17 übergeht. Diese Gestaltung hat zur Folge, dass sich ein beim Gehen und Abrollen des Fußes ergebendes seitliches Auseinanderdrücken der Schenkel 1 und 2 im Bereich der Fußsohle beschwerdelos ermöglichen lässt, indem nämlich der Steg 3 entsprechend zugelastisch nachgibt, was durch die beiden gestrichelten Anordnungen im Bereich des Steges 3 angedeutet ist, bezeichnet durch die Bereiche 3' und 3".

Figur 3 zeigt die Sprunggelenkorthese gemäß Figur 2 um 90° gedreht, wobei dem Betrachter der Schenkel 1 zugewandt sichtbar ist. Figur 3 lässt deutlich erkennen, dass vom Betrachter her gesehen hinter dem Schenkel 1 die Polsterkammer 10 angeordnet ist, die demnach am Bein des Trägers anliegt.

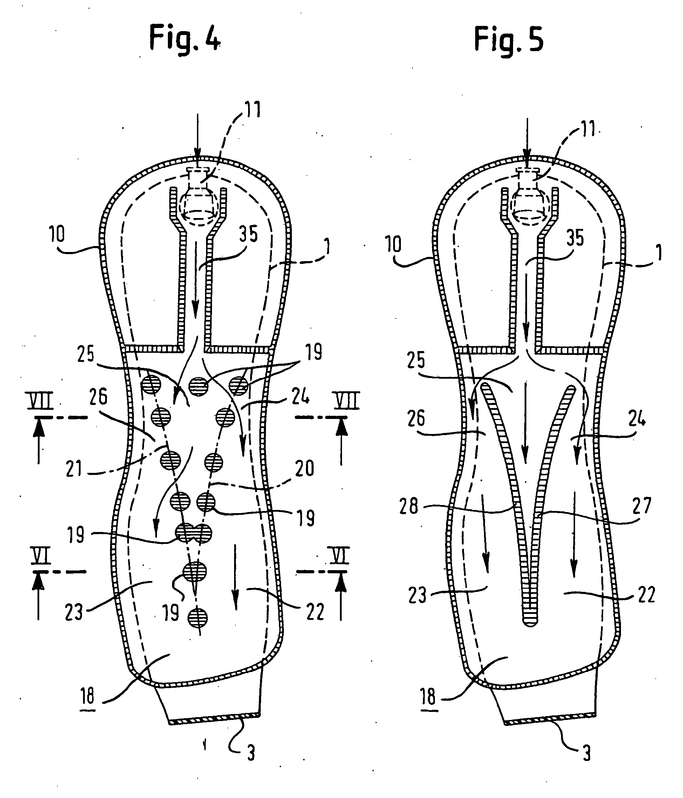

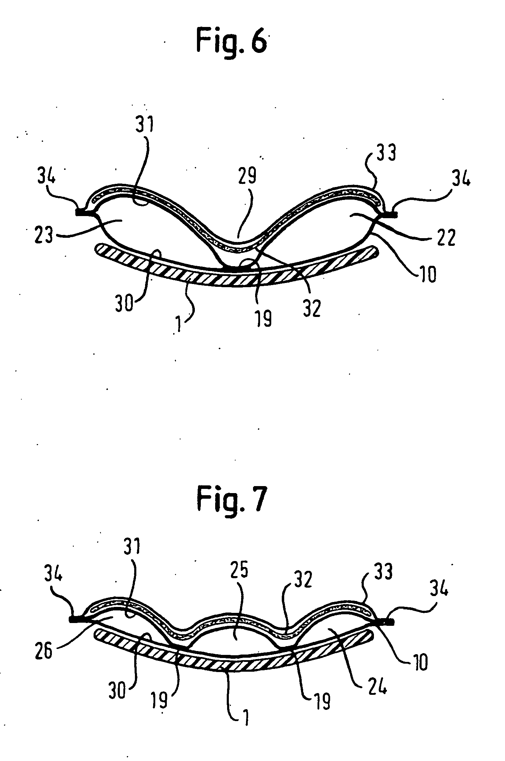

In der Figur 4 ist ein Schnitt gemäß der Linie IV-IV aus Figur 2 dargestellt, wobei also der Steg 3 im Schnitt gezeichnet ist. In der Darstellung gemäß Figur 4 zeigt sich dem Betrachter demgemäß die Innenseite der Polsterkammer 10 mit ihrem Ventil 11 und dahinter gestrichelt gezeichnet der Schenkel 1. Die Polsterkammer 10 enthält den einzigen Kammerraum 18, in den über das Ventil 11 und den Kanal 35 Luft eingeblasen wird. Der Kanal 35 wird durch entsprechend gelegte Schweißnähte gebildet. Im Bereich des Kammerraums 18 ist eine Längsunterteilung vorgesehen, gebildet durch in Reihe liegende Schweißpunkte 19, die hier V-förmig angeordnet sind und auf den strichpunktiert gezeichneten Strecken 20 und 21 liegen. Aufgrund dieser Längsunterteilung sind im Bereich des Knöchels, wo die beiden Strecken 20 und 21 zusammenlaufen, durch die Längsunterteilung zwei Unterkammern 22 und 23 gebildet, wegen der V-förmigen Erstreckung der beiden Strecken 20 und 21 drei Unterkammern 24, 25 und 26 im höheren Bereich der Sprunggelenkorthese. Wie ersichtlich, nimmt der Querschnitt dieser Unterkammern von den Unterkammern 22, 23 her zu den Unterkammern 24, 25, 26 im gefüllten Zustand kontinuierlich ab, womit bei Aufblasen der Polsterkammer 10 mit ihrem einheitlichen Kammerraum 18 eine nach oben hin geringer werdende Flächenpressung entsteht. Dort, wo die Strecken 20 und 21 zusammenlaufen, liegt etwa der Knöchel, so dass sich hier eine durch die Schweißpunkte 19 gebildete Einbuchtung als Druckentlastung für den Knöchel auswirken kann, worauf näher im Zusammenhang mit Figur 6 eingegangen wird.

In Figur 5 ist eine Variante zu der Darstellung gemäß Figur 4 gezeigt, die darin besteht, dass hier die in Reihe liegenden Schweißpunkte 19 durch die beiden Schweißnähte 27 und 28 gebildet werden, die im Effekt das Gleiche bewirken wie die auf den Strecken 20 und 21 liegenden Schweißpunkte 19.

In der Figur 6 ist ein Schnitt gemäß der Linie VI-VI aus Figur 4 dargestellt. Der Schnitt verläuft also in dem Bereich, in dem die Strecken 20 und 21 zusammenlaufen, wo auch der Knöchel der angelegten Sprunggelenkorthese liegt. Durch die dort vorhandenen Schweißpunkte 19 (bzw. die zusammengelaufenen Schweißnähte 27 und 28 gemäß Figur 5) ergibt sich die Einbuchtung 29, in die ein Knöchel hineinragen kann, ohne dort einem besonderen Druck ausgesetzt zu sein. In der Figur 6 ist noch der Schenkel 1 und die daran befestigte Polsterkammer 10 dargestellt, in der sich aufgrund des Schweißpunktes 19 (entsprechend Zusammenlaufen der Schweißnähte 27 und 28 gemäß Figur 5) eine Zusammenfügung der beiden Wände 30 und 31 der Polsterkammer 10 ergibt. Aus Figur 6 ist noch ersichtlich, dass die Polsterkammer 10 durch eine weiche Abdeckung 32 einseitig überzogen ist, die von der Haltefolie 33 in ihrer Lage an der Polsterkammer 10 festgehalten wird. Zu diesem Zweck ist die Haltefolie 33 an ihrem Rand 34 mit dem Rand der Polsterkammer 10 verschweißt. Bei dieser Gestaltung liegt also die Haltefolie 33 am Bein des Trägers der Orthese an.

Aufgrund der Anordnung des Schweißpunktes 19 ergeben sich neben diesem die beiden Unterkammern 22 und 23, die sich dann aufgrund der Längsunterteilung des gemeinsamen Kammerraums durch die beiden Strecken 20 und 21 in die Unterkammern 24, 25 und 26 fortsetzen (siehe Figur 4 und Figur 7).

In der Figur 7 ist ein Schnitt gemäß der Linie VII-VII aus Figur 4 dargestellt, der weitgehend der Darstellung in Figur 6 entspricht, allerdings mit der Änderung, dass der Schnitt VII-VII durch den Bereich der Unterkammern 24, 25 und 26 verläuft, die mit dem gemeinsamen Kammerraum 18 in Verbindung stehen und über den Zuführkanal 35 mit Luft gespeist werden.

## Patentansprüche

1. Sprunggelenkorthese mit zwei über die Knöchel den Unterschenkel entlang anzuordnenden Schenkeln (1,2), die unter dem Fuß durch einen biegsamen Steg (3) verbunden und mit ein Fluid enthaltenden Polsterkammern (10,12) versehen sind, wobei pro Schenkel (1,2) eine einzige Polsterkammer (10,12) mit einer Längsunterteilung (20,21; 27,28) vorgesehen ist, die jeweils im Bereich eines Knöchels eine Zone reduzierter Ausdehnbarkeit und damit gegenüber dem Knöchel eine diesen aufnehmende Einbuchtung (29) bildet, **dadurch gekennzeichnet, daß** der Steg (3) aus derart zugelastischem Material besteht, dass dieser beim Gehen und dabei erfolgendem Abrollen des Fußes sowie rhythmischem Pumpen der Polsterkammern (10,12) sich intermittierend verlängert und verkürzt.

2. Sprunggelenkorthese nach Anspruch 1, **dadurch gekennzeichnet, dass** die Längsunterteilung durch zwischen gegenüberliegenden Wänden (31,31) jeder Polsterkammer angeordnete Verbindungen gestaltet ist, die entlang der Längsunterteilung verlaufen.

3. Sprunggelenkorthese nach Anspruch 2, **dadurch gekennzeichnet, dass** die Verbindungen durch Verschweißen gebildet sind.

4. Sprunggelenkorthese nach Anspruch 3, **dadurch gekennzeichnet, dass** die Verbindungen durch in Reihe liegende Schweißpunkte (19) gebildet sind.

5. Sprunggelenkorthese nach Anspruch 3, **dadurch gekennzeichnet, dass** die Verbindungen durch Schweißnähte (27,28) gebildet sind.

6. Sprunggelenkorthese nach einem der Ansprüche 2 bis 5, **dadurch gekennzeichnet, dass** zwei Strecken (20, 21) von Verbindungen vorgesehen sind, die derart V-förmig verlaufen, dass die beiden Strecken im Bereich des Knöchels zusammenlaufen Und in Richtung zum Knie so weit auseinander streben, dass, ausgehend vom Bereich des Knöchels, neben diesem zwei Unterkammern (22,23) und im Übergang der Strecken in Richtung zum Knie drei nebeneinanderliegende Unterkammern (24,25,26) gebildet sind, deren Querschnitt im gefüllten Zustand abnehmend geringer ist als der Querschnitt jeder der beiden Unterkammern (22,23) im Bereich des Knöchels.

## Claims

1. Ankle brace with two arms (1, 2) arranged over the ankle bones along the lower leg, said arms (1, 2) being joined under the foot by a flexible connecting member (3) and being provided with cushioning chambers (10, 12) containing a fluid, in which each arm (1, 2) of the brace is provided with a single cushioning chamber (10, 12) with a longitudinal subdivision (20, 21; 27, 28), said longitudinal subdivision (20, 21; 27, 28) forming a zone of reduced expandability in the region of the ankle bone and therefore forming opposite the ankle bone a recess (29) which receives the latter, **characterized in that** the connecting member (3) is made of a material of such tensile elasticity that, during walking and the accompanying rolling of the foot as well as rhythmic pumping of the cushioning chambers (10, 12), said connecting member (3) is intermittently lengthened and shortened.

2. Ankle brace according to claim 1, **characterized in that** the longitudinal subdivision is formed by connections disposed between opposite walls (31, 31) of each cushioning chamber, said connections extending along the longitudinal subdivision.

3. Ankle brace according to claim 2, **characterized in that** the connections are formed by welding.

4. Ankle brace according to claim 3, **characterized in that** the connections are formed by series-arranged spot welds (19).

5. Ankle brace according to claim 3, **characterized in that** the connections are formed by welds seams (27, 28).

6. Ankle brace according to any one of claims 2 to 5, **characterized in that** two lines (20, 21) of connections are provided, said lines extending in a V-shape such that the two lines meet in the region of the ankle bone and splay so wide apart towards the knee that, starting from the region of the ankle bone, two subchambers (22, 23) are formed next to the ankle bone and three juxtaposed subchambers (24, 25, 26( are formed in the transition of the lines towards the knee, the cross section of said three juxtaposed subchambers in the inflated state being decreasingly smaller than the cross section of each of the two subchambers (22, 23) in the region of the ankle bone.

## Revendications

1. Orthèse de l'articulation de la cheville comprenant deux plaques (1, 2) qui doivent être disposées le long de la jambe au-dessus des malléoles, qui sont reliées sous le pied par une patte souple (3) et qui sont munies de compartiments de rembourrage (10, 12) contenant un fluide, dans chaque plaque (1, 2) étant prévu un compartiment de rembourrage unique (10, 12) pourvu d'un moyen de division longitudinale (20, 21 ; 27 ; 28) qui forme, dans la zone de chaque malléole, une zone à expansibilité réduite et donc, en vis-à-vis de la malléole, une cuvette (29) recevant celle-ci, **caractérisée en ce que** la patte (3) est réalisée en matériau élastique en traction, de façon à s'allonger et à se raccourcir de façon intermittente durant la marche et lors du déroulement du pied ainsi engendré de même qu'en cas de gonflage rythmique des compartiments de rembourrage (10, 12).

2. Orthèse de l'articulation de la cheville selon la revendication 1, **caractérisée en ce que** le moyen de division longitudinale est formé par des liaisons qui sont disposées entre des cloisons opposées (30, 31) de chaque compartiment de rembourrage et qui s'étendent le long du moyen de division longitudinale.

3. Orthèse de l'articulation de la cheville selon la revendication 2, **caractérisée en ce que** les liaisons sont formées par soudage.

4. Orthèse de l'articulation de la cheville selon la revendication 3, **caractérisée en ce que** les liaisons sont formées par des points de soudure (19) disposés en ligne.

5. Orthèse de l'articulation de la cheville selon la revendication 3, **caractérisée en ce que** les liaisons sont formées par des cordons de soudure (27, 28).

6. Orthèse de l'articulation de la cheville selon une des revendications 2 à 5, **caractérisée en ce qu'**il est prévu deux segments (20, 21) de liaisons qui s'étendent en V de façon à se rejoindre dans la zone de la malléole et à diverger en direction du genou pour former à partir de la zone de la malléole, à côté de celle-ci, deux sous-compartiments (22, 23) et, dans le prolongement des segments en direction du genou, trois sous-compartiments juxtaposés (24, 25, 26) dont la section transversale à l'état rempli décroît progressivement par rapport à celle de chacun des deux sous-compartiments (22, 23) situés dans la zone de la malléole.
